# EUROPEAN PATENT SPECIFICATION

(11) **EP 2 373 677 B1**
(45) Date of publication and mention of the grant of the patent: **23.03.2016**
(21) Application number: 09793497.0
(22) Date of filing: 01.12.2009
(51) Int. Cl.: C07K 1/18, C12N 9/64

(54) **POLYPEPTIDE PURIFICATION**
POLYPEPTIDREINIGUNG
PURIFICATION DE POLYPEPTIDE

(30) Priority: 02.12.2008 EP 08170454
(43) Date of publication of application: 12.10.2011
(73) Proprietor: Novo Nordisk Health Care AG, Thurgauerstrasse 36/38 8050 Zürich (CH)
(72) Inventor: BJELKE, Jais, Rose, DK-2765 Smørum (DK)
(74) Representative: Owald, Micael
(86) International application number: PCT/EP2009/066149
(87) International publication number: WO 2010/063717

(56) References cited:
- EP-A- 0 363 126
- EP-B- 0 617 049
- US-A1- 2008 207 879
- GILLIS S ET AL: "GAMMA-CARBOXYGLUTAMIC ACIDS 36 AND 40 DO NOT CONTRIBUTE TO HUMAN FACTOR IX FUNCTION" PROTEIN SCIENCE, CAMBRIDGE UNIVERSITY PRESS, CAMBRIDGE, GB, vol. 6, no. 1, 1 January 1997 (1997-01-01) , pages 185-196, XP009007552 ISSN: 0961-8368
- MALHOTRA ET AL: "Partially carboxylated prothrombins - III. Carboxylation studies" BIOCHIMICA ET BIOPHYSICA ACTA - PROTEIN STRUCTURE AND MOLECULAR ENZYMOLOGIE, ELSEVIER SCIENCE BV, AMSTERDAM, NL, vol. 746, no. 1-2, 28 July 1983 (1983-07-28), pages 81-86, XP023475965 ISSN: 0167-4838 [retrieved on 1983-07-28]
- YAN S C B ET AL: "CHARACTERIZATION AND NOVEL PURIFICATION OF RECOMBINANT HUMAN PROTEIN C FROM THREE MAMMALIAN CELL LINES" BIO/TECHNOLOGY, NATURE PUBLISHING CO. NEW YORK, US, vol. 8, no. 7, 1 July 1990 (1990-07-01), pages 655-661, XP008028568 ISSN: 0733-222X
- BURNIER J P ET AL: "GAMMA CARBOXY GLUTAMIC-ACID" MOLECULAR AND CELLULAR BIOCHEMISTRY, vol. 39, no. PART 2, 1981, pages 191-208, XP009113604 ISSN: 0300-8177
- MALHOTRA O P: "DICOUMAROL-INDUCED 9-GAMMA CARBOXYGLUTAMIC ACID PROTHROMBIN ISOLATION AND COMPARISON WITH 6-GAMMA CARBOXYGLUTAMIC ACID 7-GAMMA CARBOXYGLUTAMIC ACID 8-GAMMA CARBOXYGLUTAMIC ACID AND 10-GAMMA CARBOXYGLUTAMIC ACID ISOMERS" BIOCHEMISTRY AND CELL BIOLOGY, vol. 68, no. 4, 1990, pages 705-715, XP009113734 ISSN: 0829-8211
- OSTERUD B ET AL: "Human blood coagulation factor IX. Purification, properties, and mechanism of activation by activated factor XI." THE JOURNAL OF BIOLOGICAL CHEMISTRY 10 SEP 1978, vol. 253, no. 17, 10 September 1978 (1978-09-10), pages 5946-5951, XP002575205 ISSN: 0021-9258
- LINDSAY M ET AL: "Purification of recombinant DNA-derived factor IX produced in transgenic pig milk and fractionation of active and inactive subpopulations", JOURNAL OF CHROMATOGRAPHY, ELSEVIER SCIENCE PUBLISHERS B.V, NL, vol. 1026, no. 1-2, 13 February 2004 (2004-02-13), pages 149-157, XP004482723, ISSN: 0021-9673, DOI: 10.1016/J.CHROMA.2003.11.006

## Description

### Field of the Invention

The present invention relates to the purification of polypeptides. In particular, the present invention relates to methods utilising ion exchange chromatography, by which different gamma carboxylated forms of polypeptides can be purified.

### Background to the Invention

γ-Carboxyglutamic acid (Gla) is a unique amino acid that binds to calcium. It is a modified form of glutamic acid (Glu) and can be produced *in vivo* by the post-translational modification of glutamate residues. Carboxylation of glutamic acid in this way enables calcium binding and allows the attachment of proteins such as procoagulants and anticoagulants to phospholipids. This enzyme-mediated reaction, known as γ-carboxylation (gamma carboxylation), requires vitamin K as a cofactor.

Some mature proteins contain a domain that is rich in amino acids that have been converted to γ-carboxyglutamic acid in this way. This is known as a GLA domain. This GLA domain is often responsible for the high-affinity binding of calcium ions by the protein. Such a GLA domain may be found in a variety of different proteins. For example, blood coagulation Factors VII, IX and X and prothrombin all include a GLA domain that comprises a number of Gla amino acid residues.

Gillis S et al, Protein Science, vol. 6, no. 1, (1997), describes various human Factor IX polypeptides with different amounts of gla residues.

Malhotra et al, Biochimica et Biophysica Acta - Protein Structure and Molecular Enzymologie, vol. 746, no. 1-2, (1983) describes that prothrombins have different amounts of gla residues.

EP0363126 discloses a method for purifying vitamin-K dependent proteins, exemplified with human Protein C, using an ion exchange chromatography.

Yan S C B et al, Bio/Technology, vol. 8, no. 7 (1 July 1990) describes that the activity of Protein C is depending on the grade of gamma carboxylation.

EP0617049 describes a process for the isolation of highly purified factors IX, X and II from prothrombin complex or human plasma.

### Summary of the Invention

The present inventors have found that it is possible to separate or purify different species of a polypeptide where the different species vary in the amount of gamma carboxylation, or in the number of gamma carboxyglutamic acid residues that they contain. The invention addresses in particular the chromatographic separation of polypeptide species having different contents of gamma-carboxyglutamic acid.

Thus, the invention provides a method for purifying a polypeptide having a desired content of gamma-carboxyglutamic acid from a sample comprising mixture of species of said polypeptide having different contents of gamma-carboxyglutamic acid, said method comprising the steps of:
(a) loading said sample onto an anion exchange chromatography material;
(b) eluting said polypeptide using a solution at a pH of less than pH 9.0 comprising at least one salt selected from ammonium acetate, ammonium chloride and sodium acetate.
(c) selecting a fraction obtained from said elution wherein the polypeptides in the fraction have the desired content of gamma-carboxyglutamic acids.

A preferred such method comprises the following steps:
(a) equilibrating an anion exchange material with a buffer at a pH of less than 9.0;
(b) loading said sample onto the anion exchange material;
(c) optionally washing the anion exchange material with a buffer at a pH of less than 9.0;
(d) eluting said polypeptide from the anion exchange material using a solution at a pH of less than 9.0 comprising at least one salt selected from ammonium acetate, ammonium chloride and sodium acetate; and
(e) selecting a fraction obtained from said elution wherein the polypeptides in the fraction have the desired content of gamma-carboxyglutamic acids.

Suitable polypeptide for purification in this way include Factor IX, Factor VII, Factor VIIa, Factor X, Prothrombin, Protein-S, Protein-C, Protein Z, Osteocalcin, Matrix-gla-protein, Growth arrest-specific-6, Proline-rich-Gla-1, Proline-rich-Gla-2, Proline-rich-Gla-3 and Proline-rich-Gla-4. In a preferred method, the polypeptide is Factor IX, Factor X, Factor VII or Factor VIIa.

Where the polypeptide is Factor IX, the method may comprise selecting a fraction obtained from said elution which has an increase in the proportion of #1-11-Gla and/or #1-12-Gla forms of Factor IX compared with the proportion of #1-11-Gla and/or #1-12-Gla forms of Factor IX in the sample being purified.

Where the polypeptide is Factor X or Factor VII, the method may comprise selecting a fraction contained from said elution which has an increase in the proportion of #1-10-Gla and/or #1-11-Gla forms of Factor X or Factor VII compared with the proportion of #1-10-Gla and/or #1-11-Gla forms of Factor X or VII in the sample being purified.

Where the polypeptide is Factor IX, the method may comprise selecting a fraction obtained from said elution which has a decrease in the proportion of #1-10-Gla form of Factor IX compared with the proportion of #1-10-Gla form of Factor IX in the sample being purified.

Where the polypeptide is Factor X or Factor VII, the method may comprise selecting a fraction obtained from said elution which has a decrease in the proportion of #1-9-Gla form of Factor X or Factor VII compared with the proportion of #1-9-Gla form of Factor X or Factor VII in the sample being purified.

In the methods of the invention, any one or more of the following may apply:
- the elution buffer may have a pH of between 5.0 and 8.5;
- ammonium acetate, ammonium chloride or sodium acetate may present in the elution buffer at between 0.1 M and 2.0M, preferably at about 0.6 M.
- the ion exchange chromatography may utilise an equilibration buffer at a pH between 5.0 and 8.5.

The present invention also extends to polypeptide formulations obtained by the methods as described herein, i.e. formulations in which the amount of one or more species of the polypeptide have been altered, where the species differ in the extent of gamma carboxylation, or in the number of gamma carboxyglutamic acid residues that they contain.

### Brief Description of the Figures

Figure 1A shows the primary structure of human Factor IX with sub-domains identified. The GLA domain is found at amino acids 1-46; the EGF1 domain is found at amino acids 47-83, the EGF2 domain is found at amino acids 84 to 124, the activation peptide is found at amino acids 146 to 180 and the protease domain is found at amino acids 181 to 415. The 12 amino acids in the Gla domain that are potentially subject to gamma-carboxylation are labelled as "γ" and are located at amino acids 7, 8, 15, 17, 20, 21, 26, 27, 30, 33, 36 and 40.
Figure 1B shows an alignment of part of the amino acid sequences of the human Factor VII, Factor IX and Factor X polypeptides. These alignments are derived from the GLA domain of each of these polypeptides and show the location of Gla residues as *
Figure 2 shows the chromatogram obtained from anion exchange chromatography of a sample of rhFIX as described in Example 2. Figure 3 shows an expanded subsection of Figure 2.
Figure 4 shows an analysis of the distribution of Gla species before and after the anion exchange chromatography separation of Example 2. Before = distribution of Gla species in the sample of rhFIX used as "Application" in Example 2. This sample had been purified by immunoaffinity capture to yield a greater than 95% pure sample of rhFIX. After = distribution of Gla species in the pool of fraction C12-D3 obtained in Example 2.
Figure 5 shows the chromatogram obtained from anion exchange chromatography of a sample of rhFIX as described in Example 3. Figure 6 shows an expanded subsection of Figure 5.
Figure 7 shows the chromatogram obtained from anion exchange chromatography of a sample of FVII as described in Example 4. Figure 8 shows an expanded subsection of Figure 7.
Figure 9 shows the chromatogram obtained from anion exchange chromatography of a sample of rhFIX as described in Example 5. Figure 10 shows an expanded subsection of Figure 9.
Figure 11 shows the chromatogram obtained from anion exchange chromatography of a sample of rhFIX as described in Example 6. Figure 12 shows an expanded subsection of Figure 11.
Figure 13 shows the chromatogram obtained from anion exchange chromatography of a sample of FX as described in Example 7.

### Detailed Description of the Invention

The present invention derives from the findings that polypeptide species having different levels of gamma carboxylation may have different levels of activity and that such different species can be purified or separated using ion exchange chromatography. By increasing the proportion of more active polypeptide species, and/or by decreasing the proportion of less active or inactive polypeptide species in a sample, this can result in the production of a purified formulation having increased specific activity.

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. As used herein, the term "amino acid" refers to either natural and/or unnatural or synthetic amino acids, including glycine and both the D or L optical isomers, and amino acid analogs and peptidomimetics. A "mature" protein is a polypeptide that has been subject to post-translational processing, such as carboxylation, glycosylation or the cleavage of propeptide regions such as targeting sequences. A polypeptide of the invention that is in the form of a mature protein may thus have been subject to gamma carboxylation and may have had one or more Glu residues in its translated sequence converted to Gla.

The methods described herein may be used for the purification of any polypeptide that is, or can be, gamma carboxylated.

The polypeptide for use in the methods of the invention may be a polypeptide that comprises one or more gamma carboxyglutamic acid residues. Such a polypeptide may be identified by investigating the amino acid sequence of the polypeptide and determining whether Gla is present.

A number of polypeptides comprising gamma-carboxyglutamic acid are known. A number of blood clotting and regulatory proteins, including prothrombin, Factor VII (including Factor VIIa), Factor IX, Factor X, Protein C and Protein S, include Gla residues. These proteins can contain 10 to 12 gamma carboxyglutamic acid residues in the GLA domain, located within the first 40 residues of the N-terminus of the mature protein. Bone proteins such as osteocalcin and Matrix Gla protein and other mammalian vitamin K dependent proteins such as growth arrest-specific-6 (Gas6), Protein Z, proline-rich-Gla-1 (PRGP1), proline-rich-Gla-2 (PRGP2), proline-rich-Gla-3 (PRGP3) and proline-rich-Gla-4 (PRGP4) also comprise multiple Gla residues. Gamma carboxyglutamic acid residues have also been found in non-mammalian proteins, such as the conopeptides Conantokin G and Conantokin T. Any of these polypeptides may be purified in accordance with the invention.

The polypeptide for use in the methods of the invention may be a polypeptide that can be post-translationally modified to include one or more gamma carboxyglutamic acid residues. For example, such residues may be produced by gamma carboxylation of glutamic acid residues in the polypeptide. The amino acid sequence of the polypeptide may therefore comprise one or more glutamic acid (Glu) residues. A polypeptide that can be post-translationally modified in this way may be a naturally occurring polypeptide. Such a polypeptide may be derived from any suitable organism. For example, the polypeptide may be a naturally occurring mammalian polypeptide, such as a rodent, primate, cat, dog, sheep, cow, pig or other mammalian polypeptide. Preferably the polypeptide is a mouse, rat or human polypeptide. Most preferably the polypeptide is a human polypeptide. The polypeptide may be derived from a non-mammalian species. For example, some conotoxins can comprise gamma carboxyglutamate. The polypeptide may thus be a naturally occurring polypeptide from a mollusc such as a gastropod. The gastropod may be a snail such as a cone snail. A polypeptide that can be post-translationally modified in this way may be a variant of a naturally occurring polypeptide, such as a variant of one of the known gamma carboxylated proteins discussed above, an artificially generated polypeptide, such as a synthetic polypeptide or a recombinantly produced mutant or variant protein.

The polypeptide may be a polypeptide that has a GLA domain. A GLA domain is a protein domain that contains post-translational modifications of multiple glutamate (Glu) residues to form gamma carboxyglutamate (Gla). These Gla residues are generally located in a single region or domain of the polypeptide. This is often located at the N-terminus of the polypeptide or of the mature protein.

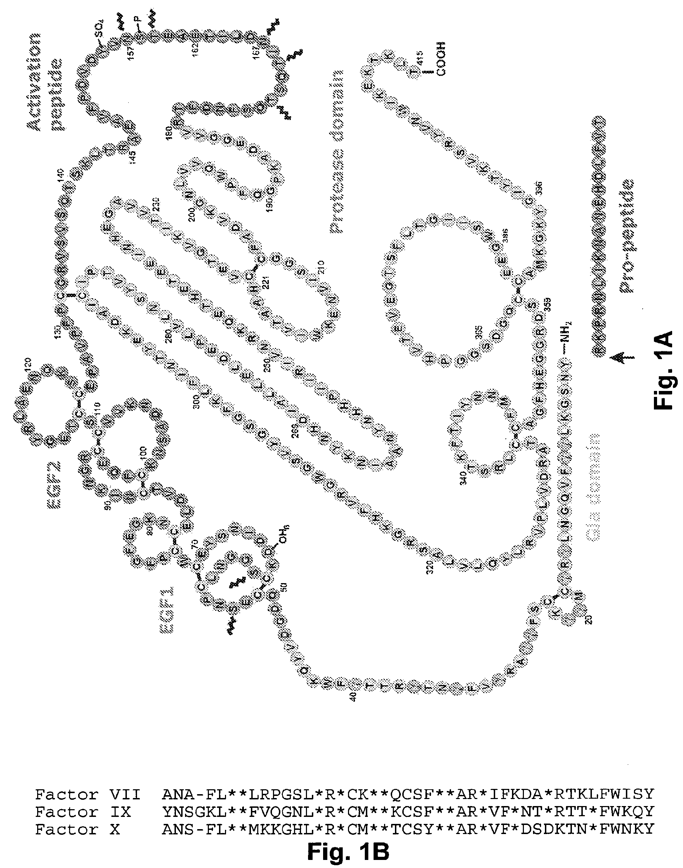

For example, Figure 1A shows the primary structure of human Factor IX protein. This protein includes a GLA domain at amino acids 1 to 46. This domain includes 12 amino acid residues that can be modified from glutamate to Gla. These are located at positions 7, 8, 15, 17, 20, 21, 26, 27, 30, 33, 36 and 40. It is therefore capable of comprising up to 12 Gla residues. The polypeptide to be purified in accordance with the present invention may therefore be Factor IX. Figure 1B shows an alignment based on the Gla domain of human Factor VII, IX and X proteins. The locations of the amino acid residues that can be modified from glutamate to Gla in each sequence are identified as*. The polypeptide to be purified in accordance with the present invention may therefore be Factor VII or Factor X.

The polypeptide for use in the invention may comprise a GLA domain from a known gamma carboxylated protein, such as a GLA domain from Factor IX, Factor VI or Factor X, or from any of the other known gamma carboxylated proteins discussed above, such as from another blood coagulation factor.

The polypeptide for use in the methods of the invention may be a polypeptide that comprises or is capable of comprising one or more gamma carboxyglutamic acid residues. For example, the polypeptide may comprise or be capable of comprising 1, 2, 3, 4, 5, 6, 7, 8, 9, 10, 11, 12, 13, 14, 15 or more Gla residues. Preferably, the polypeptide comprises or is capable of comprising more than 1 Gla residues, such as more than 1, more than 2, more than 5 or more than 9 Gla residues. The polypeptide may comprise or be capable of comprising up to 10, up to 12, up to 15 or up to 20 Gla residues. As discussed further below, the methods of the invention allow for the purification of different molecular species of the polypeptide in which different levels of gamma carboxylation have occurred. The numbers referred to here are the total number of Gla residues that may be present in that polypeptide. That is, if the polypeptide is fully gamma carboxylated, these numbers indicate the number of Gla residues that are present. For example, where gamma carboxylation takes place in the GLA domain, these numbers refer to the total number of possible sites for gamma carboxylation in that GLA domain, such as the total number of Glu residues in the translated polypeptide or the maximum number of Gla residues that may be produced by the action of an enzyme such as γ-glutamyl carboxylase. Additional species of the same polypeptide may also exist in which fewer gamma carboxyglutamic acid residues than this maximum are present.

The polypeptide may therefore comprise multiple Glu residues in the N-terminal 40 residues of its translated amino acid sequence. For example, the amino acid sequence of the expressed polypeptide may comprise 2, 3, 4, 5, 6, 7, 8, 9, 10, 11, 12 or more Glu residues in the 40 amino acids closest to the N terminus of the polypeptide, or in the 40 amino acids closest to the N terminus of the mature protein.

Gamma carboxylation may be achieved using an enzyme. Such a γ-glutamyl carboxylase enzyme is known to be involved in the gamma carboxylation of many polypeptides *in vivo.* γ-Glutamyl carboxylase is an endoplasmic enzyme which catalyses the post-translational modification of Glu into Gla in the GLA domain of a number of vitamin K dependent coagulation factors. Polypeptides for use in the present invention may thus be identified by determining whether they are gamma carboxylated by γ-glutamyl carboxylase.

The γ-glutamyl carboxylase enzyme is believed to bind to its substrate protein via a sequence motif on the amino terminal side of the glutamate residues to be carboxylated. The enzyme may then carboxylate multiple glutamate residues in that area, for example all glutamate residues in the GLA domain, before releasing the substrate. A polypeptide for use in the present invention may therefore comprise a motif or site that is recognised by γ-glutamyl carboxylase or by another enzyme capable of gamma carboxylation. This recognition site may be located in the N-terminal region of the polypeptide, for example within the 18, 19, 20, 21, 22, 23, 24, 25, 30, 35 or 40 amino acids closest to the N terminal of the polypeptide as translated, or to what will be the N terminal of the mature protein. The recognition site may be located on the amino terminal side of the glutamate residues to be carboxylated. For example, in many naturally occurring gamma carboxylated proteins, the γ-glutamyl carboxylase enzyme recognises and binds to a site in the propeptide region. That region is subsequently cleaved from the rest of the polypeptide during post-translational processing. The gamma carboxylase recognition site may therefore be absent from the mature protein.

In prothrombin and Factor IX, the site involved in recognition of the γ-glutamyl carboxylase enzyme is defined by residues -18, -17, -15, -15 and -10. A similar recognition site is found in other gamma carboxylated proteins. A phenylalanine at position -16 and alanine at position -10 are well conserved within the propeptides of carboxylase substrates, as are aliphatic residues such as isoleucine, leucine and valine at positions -17 and -15. Leucine, valine or lysine at position -16 may also support carboxylation. The polypeptide for use in the invention may comprise a gamma carboxylation recognition site from a known gamma carboxylated protein, such as a gamma carboxylation recognition site from Factor IX, Factor X, Factor VII, or any of the other known gamma carboxylated proteins discussed above. For example, a propeptide region from any such protein, which comprises a gamma carboxylation recognition site may be present at the N terminal of the translated polypeptide to allow suitable post-translational processing of the polypeptide by γ-glutamyl carboxylase.

A polypeptide capable of being gamma carboxylated preferably meets one or both of the following criteria:
(1) the polypeptide comprises a gamma carboxylation recognition site, and
(2) there are glutamic acid residues within 40 residues of the gamma carboxylation recognition site.

The γ-glutamyl carboxylase enzyme is believed to be active in the rough endoplasmic reticulum. For a polypeptide to be gamma carboxylated by this enzyme, the polypeptide preferably passes through the rough endoplasmic reticulum of a cell expressing the γ-glutamyl carboxylase enzyme. The polypeptide may thus comprise sequences allowing for the trafficking of the newly synthesised polypeptide through the rough endoplasmic reticulum. Signal sequences capable of targeting a polypeptide to the endoplasmic reticululm are well known. Such signal sequences are often found at the amino terminus of the polypeptide, may be 16 to 30 amino acids in length and may comprise 4 to 12 hydrophobic residues. Such signal peptides are generally cleaved from the polypeptide molecule by signal peptidase and so are not present in the mature protein.

In order for gamma carboxylation of the polypeptide to occur, the polypeptide is preferably expressed in a cell. A polypeptide for use in the invention may be synthesised by expression in such a cell. Preferably, the cell in which the polypeptide is expressed includes the necessary cellular machinery to allow for gamma carboxylation of a polypeptide. For example, the cell may express γ-glutamyl carboxylase. Preferably the cell in which the protein is synthesised has a gamma carboxylase enzyme associated with the rough endoplasmic reticulum. The cell may be cultured in the presence of enzyme cofactors such as vitamin K. Preferably the cell in which the protein is synthesised comprises intracellular vitamin K.

Polypeptides that are capable of being gamma carboxylated may be identified by expressing them in such a cell and determining whether gamma carboxylation occurs. For example, a polypeptide may be expressed in a cell as described herein and may be targeted to the rough endoplasmic reticulum of such a cell, for example by using a signal peptide as described above. By expressing the polypeptide in such a cell and determining whether the expressed and post-translationally processed polypeptide comprises Gla residues, a polypeptide capable of being gamma carboxylated may be identified.

The methods of the invention involve the purification of one or more species of a polypeptide from other species of that polypeptide having different degrees of gamma carboxylation. Where a polypeptide can be gamma carboxylated at more than one site, different species of that polypeptide may exist in which different numbers of gamma carboxyglutamate amino acid residues are present, or in which gamma carboxyglutamate residues are present at different possible locations within the polypeptide molecule.

For example, some species of the polypeptide may be fully gamma carboxylated. That is, gamma carboxylation may have converted glutamate to gamma carboxyglutamate at all residues in the polypeptide where this is possible, for example at all Glu residues in the GLA domain. Other species of the polypeptide may be partially gamma carboxylated. That is, gamma carboxylation may have converted glutamate to gamma carboxyglutamate at some, but not all residues in the polypeptide where this is possible, such as at some, but not all of the Glu residues in the GLA domain.

A variety of different partially gamma decarboxylated species may be identified. These may be classified in various ways. For example, the level of gamma decarboxylation may be defined by which residues in the polypeptide are gamma decarboxylated, or may be defined by the total number of gamma carboxyglutamate amino acids present in the polypeptide. The latter classification may mean that a number of structurally different molecular species of the polypeptide are considered together based on the total number of gamma carboxyglutamic acid residues that they contain. For example a species of polypeptide in which all but one of the possible gamma carboxyglutamate residues is present may contain multiple different subspecies of polypeptide, in which glutamate is retained at different positions that might have been gamma carboxylated.

Because of the mechanism of action of γ-glutamyl carboxylase, gamma carboxylation generally starts at the Glu residue closest to the gamma carboxylation recognition site and progresses away from the N terminal of the polypeptide. Where the polypeptide is not fully gamma carboxylated, this is generally because gamma carboxylation is halted, or the enzyme is released from the polypeptide, before the furthest Glu residues have been converted. It is generally the Glu residues furthest from the gamma carboxylation binding site or furthest from the N terminus of the protein that are not gamma carboxylated in a partially gamma carboxylated polypeptide.

For example, as shown in Figure 1, human Factor IX includes up to 12 gamma carboxyglutamate residues. The actual number of Gla residues present will vary in different polypeptide molecules depending upon the degree of post translational modification by gamma carboxylation that the molecule has undergone. This means that a sample of human Factor IX may comprise species of Factor IX that are fully gamma decarboxylated, i.e. that have all 12 possible gamma carboxyglutamate residues (#1-12Gla).

It may also comprise one or more species of Factor IX having 11 of the possible 12 gamma carboxyglutamate residues. Of these, the most likely is the situation where the 11 Glu residues closest to the N terminus of the polypeptide are converted to Gla, but the 12^{th} Glu residue, at position 40 as shown in Figure 1, remains as a Glu. Thus, in this situation, only Glu residues 1 to 11 have been converted to Gla (#1-11 Gla).

It may also comprise one or more species of Factor IX having 10 of the possible 12 gamma carboxyglutamate residues. Of these, the most likely is the situation where the 10 Glu residues closest to the N terminus of the polypeptide are converted to Gla, but the 11^{th} and 12^{th} Glu residues, at positions 36 and 40 as shown in Figure 1, remain as Glu. Thus, in this situation, only Glu residues 1 to 10 have been converted to Gla (#1-10Gla).

It may also comprise one or more species of Factor IX having 9 of the possible 12 gamma carboxyglutamate residues such as #1-9Gla. It may also comprise one or more species of Factor IX having less than 9, such as 8, 7, 6, 5, 4, 3, 2, 1 or none of the possible 12 gamma carboxyglutamate residues.

The present invention provides methods for the purification of such a species of polypeptide. In particular, a species of a polypeptide may be purified in relation to other species of that polypeptide in the sample. Thus, a method of the invention may lead to an increase in the relative proportion of a species of interest in the sample of the polypeptide. This may be achieved by removing one or more different species of the polypeptide from the sample, and thus increasing the proportion of the polypeptide as a whole that is formed from the species of interest. This may be achieved through specific removal of one of more particular species from the sample, by the removal of one or more species that are not the species of interest from the sample, or by removing a fraction of the sample in which the proportion of the species of interest is lower than that in the original sample. Any of these approaches may lead to an overall increase in the proportion of the species of interest. The methods of the invention may thus lead to an increase or decrease in the proportion of a particular species of polypeptide in a sample comprising a mixture of different species of that polypeptide.

An increase in the proportion of a polypeptide species may be an increase of up to 5%, up to 10%, up to 20%, up to 30% or more in the proportion of that species in the sample of the polypeptide as a whole. A decrease in the proportion of a polypeptide species may be a decrease of up to 5%, up to 10%, up to 20%, up to 30%, up to 50%, up to 70%, up to 90% or more in the proportion of that species in the sample of the polypeptide as a whole. A decrease in the proportion of a polypeptide species may be a decrease of up to 5%, up to 10%, up to 20%, up to 30%, up to 50%, up to 70%, up to 90% or more in the amount of that species that is present compared to the amount present in the original sample. A decrease in the proportion of a polypeptide species may be the complete or substantial removal of that species from the sample. For example, a method of the invention may purify a sample of a polypeptide by removing all, or substantially all, detectable polypeptide of a particular species from the sample. The amount of such a species remaining in the sample may be less than 10%, less than 5%, less than 2% or less than 1% of the amount present in the original sample.

The purpose of the methods of the invention is thus to allow the relative proportions of the different species in a sample of the polypeptide to be altered. Different species may have different properties or different activities. By changing the amount or proportion of such species in a sample of the polypeptide, the properties or activity of the sample as a whole may be altered. For example, where different species of a polypeptide have different levels of activity, by altering the proportions of different species in order to increase the proportion of species having higher levels of activity and/or by decreasing the proportions of species having lower or no activity, the specific activity of the sample, e.g. the average activity per molecule of polypeptide or the percentage of the maximum possible activity for that amount of polypeptide, may be increased.

For example, it has been found that recombinantly produced human Factor IX (rhFIX) shows a specific activity of approx. 50%. Fractionization of such a sample showed that it contained individual rhFIX species with a predominance of #1-8-, #1-9-, #1-10- #1-11- and #1-12-Gla. It has been found that #1-11- and #1-12-Gla are fully active in a clot assay and in a 2-stage activity assay. The #1-8-, #1-9- and #1-10-Gla species showed decreased activity to approx. 2-5%, 14-22% and 27-36% depending on the assay used.

It can therefore be seen that different species of rhFIX, which vary only in their degree of gamma carboxylation, show differing levels of activity. A sample with a higher proportion of #1-11- and/or #1-12-Gla would be expected to show a higher specific activity than a sample having a lower proportion of those species. A sample with a higher proportion of #1-8- and/or #1-9- and/or #1-10-Gla would be expected to show a lower specific activity than a sample having a lower proportion of those species.

Thus, the overall specific activity of a sample of Factor IX may be altered by altering the proportions of such species within the sample. In this case, it can be predicted that the overall specific activity of a sample of Factor IX may be increased by any one or more of the following:
- increasing the proportion of #1-12-Gla in the sample;
- increasing the proportion of #1-11-Gla in the sample;
- decreasing the proportion of #1-10-Gla in the sample;
- decreasing the proportion of #1-9-Gla in the sample;
- decreasing the proportion of #1-8-Gla in the sample;
- decreasing the proportion of #1-7-Gla in the sample;
- decreasing the proportion of #1-6-Gla in the sample;
- decreasing the proportion of #1-5-Gla in the sample;
- decreasing the proportion of #1-4-Gla in the sample;
- decreasing the proportion of #1-3-Gla in the sample;
- decreasing the proportion of #1-2-Gla in the sample; and
- decreasing the proportion of #1-1-Gla in the sample.

Any one or more of these changes may be selected for when purifying a sample of Factor IX in accordance with the present invention.

A preferred sample of Factor IX will comprise only #1-11-Gla and #1-12-Gla and will lack or substantially lack species having lesser degrees of gamma carboxylation than this, such as #1-10-, #1-9- and #1-8-Gla species.

This approach may be applied to existing compositions of HFIX. For example, as explained herein, the hFIX used in Example 2 was found to have 45.6% #1-12-Gla, 36.0% #1-11-Gla, 13.6% #1-10-Gla, 3.3% #1-9-Gla and 1.6% #1-8-Gla. rhFIX is commercially available from Wyeth under the mark Benefix®. Benefix® has a #1-10-, #1-11- and #1-12-Gla content of approx. 8-10%, 25-31% and 60-67%, respectively. It can be seen that the methods described herein may be used to increase the proportion of #1-11-Gla and/or #1-12-Gla in such compositions. The methods described herein may be used to decrease the proportion of less gamma carboxylated species such as #1-10-Gla, #1-9-Gla and #1-8-Gla in such formulations. This would be expected to improve the specific activity of the hFIX in such a formulation.

A similar approach may be used in relation to other Gla-containing polypeptides. For example, Factor VII and Factor X may include up to 11 Gla residues. As shown in Example 4, the methods of the present invention may be used to alter the proportions of, for example, #1-9-, #1-10-, or #1-11- Factor VII. The methods as described herein may thus be used to alter the proportions of different species of Factor VII depending upon their Gla content. For example, the methods described herein may be used to increase the proportion of #1-10-Gla and/or #1-11-Gla in such compositions. The methods described herein may be used to decrease the proportion of less gamma carboxylated species, for example #1-9-Gla and lower, in such compositions.

Similarly, Example 7 shows that a formulation comprising multiple species of Factor X may be processed according to a method of the invention in order to alter the proportions of different Gla-containing species of Factor X. By selecting suitable fractions during anion exchange chromatography, samples comprising different proportions of different Gla species may be selected. For example, it can be seen from Example 7 that the methods described herein may be used to increase the proportion of #1-10-Gla and/or #1-11-Gla in such compositions. The methods described herein may be used to decrease the proportion of less gamma carboxylated species such as #1-9-Gla and lower.

The invention thus provides methods which allow a species of a polypeptide to be purified from other species of the same polypeptide, wherein the species differ in the extent to which they are gamma carboxylated, or in the number of Gla residues in their amino acid sequence.

The methods of the invention use anion exchange chromatography. The invention relates in particular to methods where a polypeptide of interest is bound to an anion exchange material and elution of the polypeptide from the anion exchange material is performed using a buffer that comprises ammonium acetate, ammonium chloride or sodium acetate.

An anion exchange material is an ion exchange material that is positively charged. It therefore has free anions that can be exchanged with anions in an aqueous solution passed over or through the anion exchange material. The charge may be provided by attaching one or more charged ligands to the solid phase or it may be an inherent property of the solid phase. The solid phase may be, for example, a purification column, particles or beads, a membrane or a filter. In general an anion exchange resin may be used for the purification of polypeptides with a pl of less than about 7. Commercially available anion exchange materials that may be used as described herein include, for example, Q Sepharose Fast Flow, Macroprep 25Q, Poros HQ50, Source 30Q, Source 15Q, Mini Q, Mono Q, preferably Mini Q, Mono Q, Capto Q, Q Sepharose HP, Toyopearl QAE 550C, Unosphere Q, DE-AE Sepharose FF, Fratoprep DEAE and Q HyperD 20.

An anion exchange material may utilise a strong anion exchange group such as a quaternary amine. An anion exchange material may utilise a weak anion exchange group such as diethylaminoethyl (DEAE). A suitable anion exchange material may be selected by the skilled artisan depending upon, for example, the particular polypeptide to be purified.

The anion exchange material may be selected depending upon the specific polypeptide to be purified and the conditions employed such as pH, buffer, ionic strength etc.

A conventional anion exchange chromatography purification process usually consists of one or more steps selected from: equilibration of the anion exchange material, application or loading of a sample, one or more washing steps, elution and regeneration of the ion exchange material. Standard methods for ion exchange chromatography may be found in, for example, Remington's Pharmaceutical Sciences.

The anion exchange resin is preferably equilibrated prior to loading the polypeptide of interest. The purpose of this equilibration step is to adjust the conditions at the anion exchange material to more closely resemble those to be used in the subsequent steps of the method. To avoid changes in the composition of the mobile phase during the chromatography, the anion exchange material should be equilibrated to the pH and ionic composition (e.g. conductivity, buffer composition) of the starting buffer. For example, the ionic strength (e.g. conductivity, pH) of the equilibration buffer may be selected to be as similar as possible to the ionic strength of buffers to be used in later steps of the method, such as the buffer used to load to polypeptide and/or the wash buffer(s).

The anion exchange material may therefore be equilibrated using a buffer that is closely based on the buffers or formulations to be used in the subsequent steps. For example, the same buffer may be used for equilibration of the anion exchange material and for loading the sample. The same buffer may be used for equilibration of the anion exchange material and for washing the anion exchange material after the sample has been loaded. The equilibration buffer may have the same pH as the loading formulation and/or the wash buffer. The equilibration buffer may have the same conductivity as the loading formulation and/or the wash buffer. The equilibration buffer may use the same buffering substance as the loading formulation and/or the wash buffer. The equilibration buffer may have the same buffering substance concentration as the loading formulation and/or the wash buffer. The equilibration buffer may comprise additional components also present in the loading formulation and/or the wash buffer, such as detergents.

The pH of the equilibration buffer may be determined depending on the particular polypeptide to be purified. For example, for a number of polypeptides such as Factor IX, a pH of 9.0 or higher is not optimal, since autoactivation and/or degradation of the polypeptide may be observed at these pH values.

An equilibration buffer for use in the present invention may be formulated at, for example at pH of from about 5.0 to about 8.5, such as from pH 5.0 to pH 8.5. The pH of an equilibration buffer may be greater than about 5.0, greater than about 5.5, greater than about 6.0, greater than about 6.5, greater than about 7.0, greater than about 7.5 or greater than about 8.0. The pH of the equilibration buffer may be less than about 8.5, less than about 8.0, less than about 7.5, less than about 7.0, less than about 6.5, less than about 6.0 or less than about 5.5. Any combination of such end points may be combined. For example the pH of the equilibration buffer may be greater than about 7.0 and less than about 8.5. The pH may be, for example, about pH 7.0, 7.5, 8.0 or 8.5.

These pH values may be suitable for the equilibration of anion exchange materials for the purification of polypeptides as described herein, such as Factor IX, Factor VII or Factor X.

Suitable components for an equilibration buffer may include a buffering substance, e.g. Tris, phosphate, MES, Hepes or carbonate. For an anion exchange chromatography method, a positive buffering ion such as Tris is preferred. Such a buffering substance may be used to maintain the equilibration buffer at a pH as defined above. In one embodiment, the same buffering substance and buffering substance concentration are used throughout the anion exchange chromatography procedure. For example, the equilibration buffer, wash buffer(s) and elution buffer may all comprise the same buffering substance at the same buffering substance concentration. The buffering substance concentration should be sufficient to maintain buffering capacity and constant pH during the anion exchange procedure. For example, the buffering substance and buffering substance concentration may be selected to maintain a stable pH and buffering capacity during application of the sample and during elution. A suitable buffering substance concentration may be, for example, between 5mM and 50mM, such as between 10 mM and 40mM. A suitable buffering substance concentration may be, for example, 5mM, 10mM, 15mM, 20mM or 25mM.

An equilibration buffer may comprise one or more additional components. An equilibration buffer may comprise an additive such as ethylene glycol, ethanol, urea or a detergent used to increase solubility of a protein. A detergent used in anion exchange chromatography should be neutral or of the same charge as the anion exchange material. Non-ionic detergents such as Tween 80, Tween 20 or Triton X100 may be used in a concentration of, for example, less than 1%, less than 0.5%, less than 0.1% or less than 0.01%. A non-buffering salt, such as NaCl may be used to adjust the ionic strength of the buffer.

A sample comprising the polypeptide of interest is loaded onto the anion exchange material. This is achieved by exposing the sample to the anion exchange material under appropriate conditions (such as conductivity and/or pH) such that the polypeptide is immobilised in or on the anion exchange material. This immobilisation or binding is achieved by ionic interactions between the polypeptide and charged groups of the anion exchange material. Such binding generally occurs when the ionic strength of the mobile phase in contact with the anion exchange material is reduced to the point that the ionic groups of the polypeptide of interest begin to serve as the counter ions for the charged groups on the anion exchange material.

The sample to be purified in a method of the invention may be any sample comprising a polypeptide as described above. Preferably the sample comprises more than one different species of the same polypeptide wherein the species vary in the degree and/or location of their gamma carboxylation.

As mentioned above, the polypeptide of interest may be obtained using any routine procedure. For example, the polypeptide may be obtained from an *in vivo* source, such as from an animal, or may be produced *in vitro,* for example in a tissue or cell. The polypeptide of interest may be recombinantly produced, for example by inducing the expression of the polypeptide in a cell. For example, the polypeptide of interest may be produced in a host cell that has been transformed or transfected with a polynucleotide that encodes, and is capable of expressing, the polypeptide. Such a host cell may be cultured under conditions that allow the expression of the polypeptide. The polypeptide may then be recovered from the culture medium or from the host cells themselves.

Preferably the polypeptide is purified before being applied to the anion exchange resin. For example, the polypeptide may be subjected to one or more purification steps such as precipitation, immunoprecipitation, isoelectric focusing, filtration, centrifugation or chromatography such as other anion exchange chromatography.

Such purification may be used to remove, partially or totally, one or more contaminants from the sample and thereby increase the degree of purity of the polypeptide of interest. The contaminant may be any molecule that is not the polypeptide of interest. For example, the contaminant may be a different polypeptide, a nucleic acid or an endotoxin. The contaminant may be a variant of the polypeptide of interest, such as a truncated or extended polypeptide, a deamidated form of the polypeptide, an incorrectly folded polypeptide or a form of the polypeptide having undesired glycosylation. The contaminant may be a molecule that might interfere with the ion exchange chromatography.

Preferably the polypeptide of interest is at least 75% pure, more preferably at least 80%, at least 90% or more. Most preferably the polypeptide is at least 90% pure, such as at least 95%, at least 97% or at least 99% pure. Purity is intended to refer to the proportion of the total dry weight that is made up of the polypeptide of interest. The sample may comprise less than 25% by weight of contaminants as described above, such as less than 25% by weight of proteins other than the polypeptide of interest, more preferably less than 20%, less than 10%, less than 5%, less than 3% or less than 1%. The sample may be a pure or substantially pure sample of the polypeptide of interest. The sample may be an isolated or substantially isolated sample of the polypeptide of interest.

Such a sample of polypeptide may be applied to the anion exchange material in a form obtained directly from the polypeptide synthesis, such as in the form of a sample from the culture medium of cells that recombinantly produce the polypeptide or a sample of lysed cells that expressed the polypeptide. A sample of polypeptide may be applied to the anion exchange material in a purified or partly purified form as described herein. A sample as described herein may be further formulated before application to the anion exchange material. For example, where the polypeptide (or purified polypeptide) is provided in a solid form, it may be formulated in a liquid composition for application to the anion exchange material. For example, it may be formulated in water, a buffer or another solvent. Preferably, the liquid composition is aqueous. Where the polypeptide or purified polypeptide is provided in a liquid or aqueous form, or where a solid polypeptide sample has been formulated in a liquid form as described above, the formulation of the sample may be adjusted before it is applied to the anion exchange material.

For example, the conductivity and/or the pH of the sample or formulated sample may be adjusted using routine methods. The pH of the sample may be adjusted to be the same as, or substantially the same as, that of the buffers used for equilibration of the anion exchange material and/or washing of the anion exchange material. The conductivity of the sample may be adjusted to be the same as, or substantially the same as, that of the buffers used for equilibration of the anion exchange material and/or washing of the anion exchange material. The sample may be formulated with a buffering substance, such as any of the buffering substances discussed above in relation to the composition of the equilibration buffer. The sample may be formulated in the same buffer used for equilibration of the anion exchange material and/or washing of the anion exchange material. The sample may be formulated in the same buffering substance and/or the same buffering substance concentration and/or the same pH and/or the same conductivity as the buffer used for equilibration of the anion exchange material and/or washing of the anion exchange material. In one embodiment, a polypeptide of interest is formulated for application to an anion exchange material by adding it to a buffer identical to the equilibration buffer that is being used.

The polypeptide is then loaded onto the ion exchange material by passing the relevant formulation of the polypeptide over or through the anion exchange material under conditions that allow for binding of the polypeptide to the anion exchange material. Such methods are routine in the art.

Once the polypeptide of interest is loaded onto the anion exchange column, the column may be subjected to one or more washes. Washing is achieved by passing an appropriate solution through or over the anion exchange material. The purpose of such washes may include to remove any polypeptide or other components that are not bound to the anion exchange material; to remove any polypeptide or other components that are only weakly bound to the anion exchange material; to remove impurities that bind to the anion exchange material with a lower affinity than the polypeptide of interest.

In one embodiment, after loading of the polypeptide onto the anion exchange material, the anion exchange material is washed with a buffer in order to remove any unbound polypeptide, contaminants or impurities. For example, the wash buffer may be identical to, or substantially identical to, the buffer in which the polypeptide was formulated for loading onto the anion exchange material. The wash buffer may be identical to, or substantially identical to, the equilibration buffer. For example, a wash may be carried out using the same buffer as the equilibration buffer or the same buffer used to formulate the polypeptide. A wash may be carried out using a buffer having the same or substantially the same pH and/or the same or substantially the same conductivity as the equilibration buffer or the buffer used to formulate the polypeptide. A wash may be carried out using a buffer that comprises the same buffering substance at the same or substantially the same concentration as that used in the equilibration buffer or for the formulation of the polypeptide.

Other washes may alternatively or additionally be carried out using buffers that are different to the equilibration buffer. For example, it may be possible to remove contaminants from the anion exchange material that bind to the anion exchange material less strongly than the polypeptide of interest. Such contaminants will be released from the anion exchange material more easily than the polypeptide of interest. For example, the anion exchange material may be washed with a buffer having a greater conductivity or ionic strength than the equilibration buffer and/or the formulation in which the polypeptide was loaded. By increasing the ionic strength of the buffer, elution of components from the anion exchange material may be achieved. Preferably the wash buffer is selected, or is used in a sufficiently small volume such that substantially no polypeptide of interest is eluted from the anion exchange material.

Buffers for washing may be selected by a skilled artisan depending upon the nature of the particular sample and polypeptide of interest. For example, buffers may be selected having a particular pH or conductivity to allow for the removal of particular polypeptides or impurities that will bind to the resin less strongly than the polypeptide of interest. Such buffers may be selected and their use optimised by simple routine experiments, for example by monitoring the composition of the solution removed from the column.

The pH of a wash buffer may be determined depending on the particular polypeptide to be purified. For example, for a number of polypeptides, such as Factor IX, a pH of 9.0 or higher is not optimal, since autoactivation and/or degradation of the polypeptide may be observed at these pH values.

A wash buffer suitable for use in the present invention may be formulated at, for example at pH of from about 5.0 to about 8.5, such as from pH 5.0 to pH 8.5. The pH of a wash buffer may be greater than about 5.0, greater than about 5.5, greater than about 6.0, greater than about 6.5, greater than about 7.0, greater than about 7.5 or greater than about 8.0. The pH of the wash buffer may be less than about 8.5, less than about 8.0, less than about 7.5, less than about 7.0, less than about 6.5, less than about 6.0 or less than about 5.5. Any combination of such end points may be combined. For example the pH of the wash buffer may be greater than about 7.0 and less than about 8.5. The pH may be, for example, about pH 7.0, 7.5, 8.0 or 8.5.

These pH values may be suitable for the washing of anion exchange materials for the purification of polypeptides as described herein, such as Factor IX, Factor VII or Factor X.

Suitable components for a wash buffer may include a buffering substance, e.g. Tris, phosphate, MES, Hepes or carbonate. For an anion exchange chromatography method, a positive buffering ion such as Tris is preferred. Such a buffering substance may be used to maintain the wash buffer at a pH as defined above. A suitable buffering substance concentration may be, for example, between 5mM and 50mM, such as between 10 mM and 40mM. A suitable buffering substance concentration may be, for example, 5mM, 10mM, 15mM, 20mM or 25mM.

A wash buffer may comprise one or more additional components. A wash buffer may comprise an additive such as ethylene glycol, ethanol, urea or a detergent used to increase solubility of a protein. A detergent used in anion exchange chromatography should be neutral or of the same charge as the anion exchange material. Non-ionic detergents such as Tween 80, Tween 20 or Triton X100 may be used in a concentration of, for example, less than 1%, less than 0.5%, less than 0.1% or less than 0.01%. A non-buffering salt, such as NaCl may be used to adjust the ionic strength of the buffer.

To elute a molecule from an anion exchange material is meant to remove the molecule from the anion exchange material. This is generally achieved by altering the ionic strength of the buffer surrounding the anion exchange material so that the buffer competes with the molecule for the charged groups of the anion exchange material. The binding strength of the molecule for the anion exchange material therefore decreases and it detaches. Elution from an anion exchange material may also be achieved in some cases by using a molecule that alters the conformation of the polypeptide of interest, thus reducing the binding strength and causing the polypeptide to be released from the anion exchange material.

The pH of the elution buffer may be determined depending on the particular polypeptide to be purified. For example, for a number of polypeptides, such as Factor IX, a pH of 9.0 or higher is not optimal, since autoactivation and/or degradation of the polypeptide may be observed at these pH values.

An elution buffer suitable for use in the present invention may be formulated at, for example at pH of from about 5.0 to about 8.5, such as from pH 5.0 to pH 8.5. The pH of an elution buffer may be greater than about 5.0, greater than about 5.5, greater than about 6.0, greater than about 6.5, greater than about 7.0, greater than about 7.5 or greater than about 8.0. The pH of the elution buffer may be less than about 8.5, less than about 8.0, less than about 7.5, less than about 7.0, less than about 6.5, less than about 6.0 or less than about 5.5. Any combination of such end points may be combined. For example the pH of the elution buffer may be greater than about 7.0 and less than about 8.5. The pH may be, for example, about pH 7.0, 7.5, 8.0 or 8.5.

These pH values may be suitable for the elution of anion exchange materials for the purification of polypeptides as described herein, such as Factor IX, Factor VII or Factor X.

Suitable components for an elution buffer may include a buffering substance, e.g. Tris, phosphate, MES, Hepes or carbonate. For an anion exchange chromatography method, a positive buffering ion such as Tris is preferred. Such a buffering substance may be used to maintain the elution buffer at a pH as defined above. A suitable buffering substance concentration may be, for example, between 5mM and 50mM, such as between 10 mM and 40mM. A suitable buffering substance concentration may be, for example, 5mM, 10mM, 15mM, 20mM or 25mM.

An elution buffer may comprise one or more additional components. An elution buffer may comprise an additive such as ethylene glycol, ethanol, urea or a detergent used to increase solubility of a protein. A detergent used in anion exchange chromatography should be neutral or of the same charge as the anion exchange material. Non-ionic detergents such as Tween 80, Tween 20 or Triton X100 may be used in a concentration of, for example, less than 1%, less than 0.5%, less than 0.1 % or less than 0.01%. A non-buffering salt, such as NaCl may be used to adjust the ionic strength of the buffer.

For use in accordance with the present invention, the elution buffer will preferably comprise one or more chaotropic salts, such as one or more salts selected from ammonium acetate, ammonium chloride and sodium acetate. The chaotropic salt such as ammonium acetate, ammonium chloride and/or sodium acetate may be present in the elution buffer at a concentration of at least 0.1 M, at least 0.2M, at least 0.5M, at least 1.0M or at least 1.5 M. The chaotropic salt such as ammonium acetate, ammonium chloride and/or sodium acetate may be present at a concentration of up to 2.0M, up to 1.9M, up to 1.5M or up to 1.0M. Any one of these lower end points may be combined with any one of these upper end points to form a suitable concentration range. The chaotropic salt such as ammonium acetate, ammonium chloride and/or sodium acetate may be present at any concentration up to about 2.0M. For example, the chaotropic salt such as ammonium acetate, ammonium chloride and/or sodium acetate may be present at a concentration of up to about 0.1, about 0.2, about 0.3, about 0.4, about 0.5, about 0.6, about 0.7, about 0.8, about 0.9, about 1.0, about 1.1, about 1.2, about 1.3, about 1.4, about 1.5, about 1.6, about 1.7, about 1.8, about 1.9 or about 2.0 M, most preferably at a concentration of about 0.6 M.

In one embodiment the elution buffer has the same composition as a wash buffer and/or the equilibration buffer except that the elution buffer additionally comprises chaotropic salt. A preferred chaotropic salt is one or more of ammonium acetate, ammonium chloride and/or sodium acetate as described above. Thus, the elution buffer may have any composition described herein for a wash buffer or equilibration buffer, but may additionally comprise ammonium acetate, ammonium chloride and/or sodium acetate.

In one embodiment, the equilibration buffer and wash buffer are identical and the elution buffer differs from them only in that the elution buffer also comprises ammonium acetate, ammonium chloride or sodium acetate

Elution may be performed using an isocratic or linear gradient of the chaotropic salt, such as isocratic or linear gradient of ammonium acetate, ammonium chloride or sodium acetate. Elution may be performed using a step-wise change in the concentration of the chaotropic salt in the buffer. Elution may be achieved by any combination of these elution approaches. For example, isocratic elution at a given concentration of chaotropic salt may be followed by an increase in concentration of the salt either in the form of a gradient or one or more steps.

In any such elution method, different components will be released from the anion exchange material at different times, depending upon the strength of their binding. Components that bind less strongly will tend to be released earlier or at a lower buffer conductivity such as a lower salt concentration. Components that bind more strongly will tend to be retained on the anion exchange material for longer or at a higher salt concentration. The eluant that has passed across or through the anion exchange material may be monitored to identify when particular components are eluted. The eluant may be pooled at different time points and each pool analysed to determine which components are present in which pools. Particular pools may then be selected that have the desired formulation, for example increased concentrations of particular polypeptide species or decreased concentrations of other polypeptide species.

Isocratic elution as described herein uses a fixed or steady concentration of the salt. An elution buffer is used which comprises that concentration of the salt, such as any of the concentrations discussed above. The elution buffer is passed across or through the anion exchange material and the eluant is monitored to identify when elution occurs. Using isocratic elution, components having lower binding affinity for the anion exchange material will be released earlier, when a smaller volume of elution buffer has been used, than components having a higher binding affinity, which may require greater volumes of elution buffer to be passed across or through the anion exchange material. By selecting particular pools or batches of eluant obtained at different time periods, samples that have different compositions of polypeptide species may be obtained.

Gradient elution may be achieved by increasing the concentration of the salt in the buffer up to a final maximum concentration, such as a concentration as discussed above. For example a linear gradient may use from 0% to 100% of the final concentration of the chaotropic salt. This gradient may be applied to the anion exchange material over a period of time, such as over 10, 20, 30 40, 50, 70, 100, 150 or more column volumes. Using such gradient elution, components having lower binding affinity for the anion exchange material will be released earlier, at a lower concentration of salt, than components having a higher binding affinity, which may require a higher concentration of salt for elution to occur. By selecting particular pools or batches of eluant obtained at different time periods, samples that have different compositions of polypeptide species may be obtained.

Rather than using a gradual gradient to increase the concentration of the chaotropic salt, a stepwise increase may be used. That is, the concentration of the salt may be increased to a final maximum concentration in one or more discrete steps. This may be used to mirror the effects of a gradient elution, wherein different components are released at different concentrations and thus different steps. Stepwise elution may alternatively be combined with isocratic elution. For example, a stepped increase in salt concentration may be maintained for a number of column volumes of the elution buffer, such that isocratic elution at that concentration is allowed to occur, with different components being eluted as increasing volumes of the buffer are used. Subsequent additional steps in salt concentration may also be used.

In the embodiments where the elution buffer differs from the wash buffer only in the presence of a salt such as ammonium acetate, ammonium chloride or sodium acetate in the elution buffer, an elution buffer for isocratic elution may be obtained by adding an amount of the salt to the wash buffer, gradient elution may be achieved by gradually adding the salt to the wash buffer solution and a stepwise elution may be achieved by adding amounts of the salt to the wash buffer in order to increase the concentration of the salt in the buffer in discrete steps.

### Examples

### Example 1: Analysis of activity of different gamma-carboxylated species of FIX.

Recombinant human Factor IX (FIX) was produced in Chinese hamster ovary (CHO) cells. The specific activity of the FIX was measured to be approximately 50%.

Individual FIX species with a predominance of #1-8-, #1-9-, #1-10-, #1-11- and #1-12-Gla were fractionated.

A clot assay was used to measure FIX activity-dependent time to fibrin clot formation. A contact activator (ellagic acid in the APTT reagent) was used to stimulate the production of FXIIa and was further triggered by re-calcification and added phospholipids. Activities were measured against BeneFIX, and normal pooled human plasma, which was calibrated against WHO human FIX standard.

A commercially available assay kit known as 'Hyphen BioMed Chromogenic Factor IX kit (Aniara)' was used to assess the activity level of rhFIX. In this assay, Factor Xla activates Factor IX into Factor IXa, which together with activated Factor VIII:C, phospholipids and Ca²⁺, activates Factor X into Factor Xa. The amount of generated Factor Xa was measured at 405nm by the amount of pNA released from the Factor Xa specific chromogenic substrate SXa-11.

It was found that the #1-11- and #1-12-Gla species were fully active in clot and 2-stage activity assays. The #1-8-, #1-9- and #1-10-Gla species dropped in activity to approximately 2-5%, 14-22% and 27-36% respectively, depending upon the assay used.

### Example 2: Purification and analysis of different gamma-carboxylated species of FIX

Anion exchange chromatography was used for the separation of different gamma carboxylated forms for recombinant human factor IX (rhFIX). The method used a SOURCE 15Q column (GE Healthcare) with a bed volume of 6 ml, a flow rate of 3 ml/min and a temperature of 4°C.

The sample loaded to the column ("loading sample") was of rhFIX. A total of approx. 13 mg was loaded. The loading sample was adjusted to pH 8.0 using 0.1 M NaOH.

The buffers used were as follows:
Equilibration buffer: 20 mM Tris/NaOH, pH 8.0, 0.01 % Tween80.
Elution buffer: 20 mM Tris/HCl, 1.5 M Ammonium acetate, pH 8.0, 0.01 % Tween80.
CIP: 1 M NaOH

The anion exchange chromatography procedure was as follows:

| **Step** | **Buffer** | **CV** | | **%-elution buffer** |
|---|---|---|---|---|
| Equilibrate | Equilibration buffer | 10 | | 0% |
| Application | | | | |
| Wash | Equilibration buffer | 5 | | 0% |
| Elution | Elution buffer | 5 | | 20 % (isocratic) |
| Elution | Elution buffer | 20 | | 20-40% (gradient) |
| Elution | Elution buffer | 5 | | 65% (isocratic) |
| Elution | Elution buffer | 5 | | 100% (isocratic) |
| CIP1 | NaOH | 5 | | 0% |
| CIP2 | Elution buffer | 10 | | 100% (isocratic) |
| Reequilibrate | Equilibration buffer | 20 | | 0% |

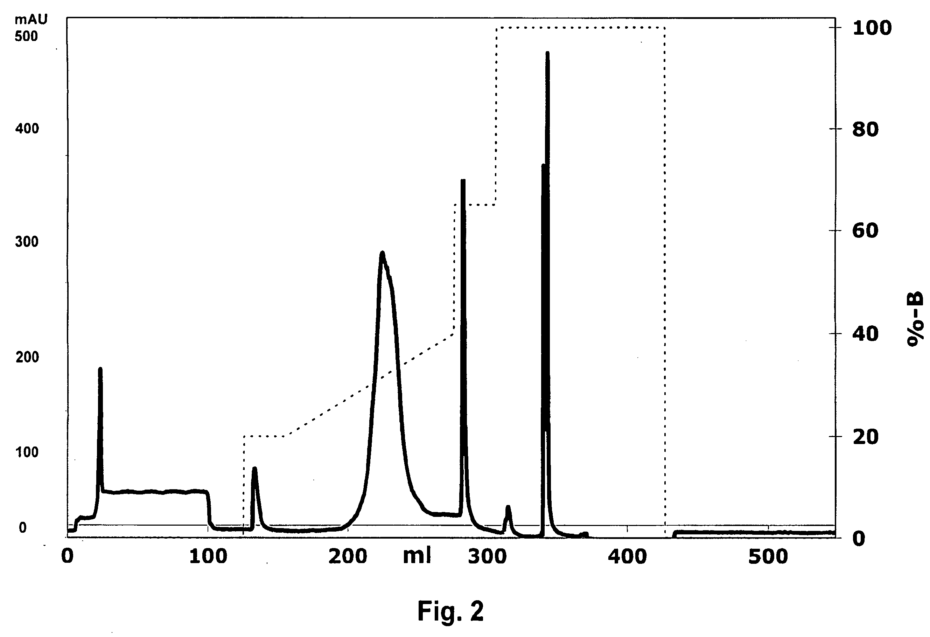

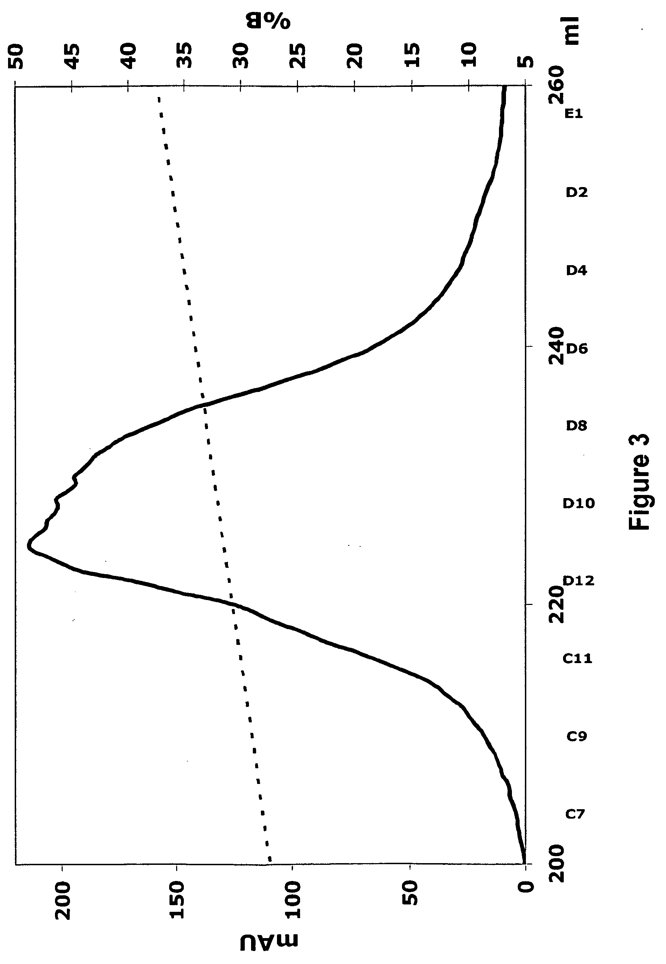

The results of this anion exchange chromatography are shown in Figure 2 and 3.

A pool of fraction C12-D3 was chosen, yielding 9.6 mg/74 %. The Gla content of this pool was compared with that of the original sample used for loading sample above.

Gla content analyses of individual fractions were performed using an Agilent HPLC system. The Gla content analyses based on the HPLC method correlated with Gla content analyses obtained using N-terminal sequencing and amino acid basic hydrolysis analyses. The HPLC used a MiniQ PC3.2/3 column (GE Healthcare cat. no 17-0686-01) at a flow rate of 0.18 ml/min. The buffers used in this system were:
A-buffer: 20 mM Tris/NaOH, pH 9.0
B-buffer: 20 mM Tris/HCl, pH 9.0, 1.5 M Ammonium acetate

The signals measured in this system were UV280 and fluorescence signal (ex: 280nm / em: 340nm).

The HPLC procedure was as follows:

| | |
|---|---|
| 0 - 5 min. | 0 - 30 % B |
| 5 - 55 min. | 30 - 55 % B |
| 55 - 65 min. | 55 - 100 % B |

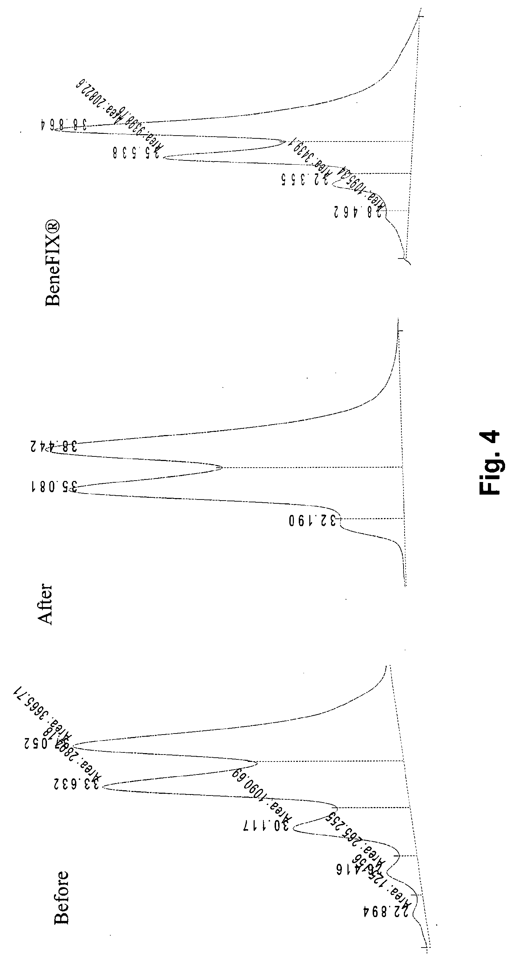

A comparison of the distribution of Gla before and after separation using anion exchange chromatography as described above is shown in Figure 4. These results may also be presented as follows.

| **%-#Gla** | **Before** | **After** | **BeneFIX®, incl. for comparison** |
|---|---|---|---|
| %-#1-8 | 1.6 | 0 | 0 |
| %-#1-9 | 3.3 | 0 | 3 |
| %-#1-10 | 13.6 | 6.7 | 10 |
| %-#1-11 | 36.0 | 40.7 | 27 |
| %-#1-12 | 45.6 | 52.6 | 60 |

Purification using anion exchange chromatography as shown above thus led to the removal of all detectable #1-8- and #1-9-Gla from the original FIX sample. There was also a decrease in the presence of #1-10-Gla and a consequent increase in the proportions of #1-11- and #1-12-Gla.

In the final purified pool the specific activity also increased to approx. 110 % compared to Benefix (2-stage activity assay, data calculations not shown).

### EXAMPLE 3: Purification and analysis of different gamma-carboxylated species of FIX

Anion exchange chromatography was used for the separation of different gamma carboxylated forms for recombinant human FIX. The method used a SOURCE 30Q column (GE Healthcare) with a bed volume of 6 ml, a flow rate of 2 ml/min and a temperature of 4°C.

The sample loaded to the column ("loading sample") was of FIX. A total of approx. 2.5 mg was loaded. The loading sample was adjusted to pH 8.0 using 0.1 M NaOH.

The buffers used were as follows:
Equilibration buffer: 20 mM Tris/NaOH, pH 8.0, 0.01 % Tween80.
Elution buffer: 20 mM Tris/HCl, 1.5 M Ammonium acetate, pH 8.0, 0.01 % Tween80.
CIP: 1 M NaOH

The anion exchange chromatography procedure was as follows:

| **Step** | **Buffer** | **CV** | | **%-elution buffer** |
|---|---|---|---|---|
| Equilibrate | Equilibration buffer | 10 | | 0% |
| Application | | | | |
| Wash | Equilibration buffer | 5 | | 0% |
| Elution | Elution buffer | 5 | | 25 % (isocratic) |
| Elution | Elution buffer | 20 | | 25-45% (gradient) |
| Elution | Elution buffer | 5 | | 100% (isocratic) |
| CIP1 | NaOH | 5 | | 0% |
| CIP2 | Elution buffer | 10 | | 100% (isocratic) |
| Reequilibrate | Equilibration buffer | 20 | | 0% |

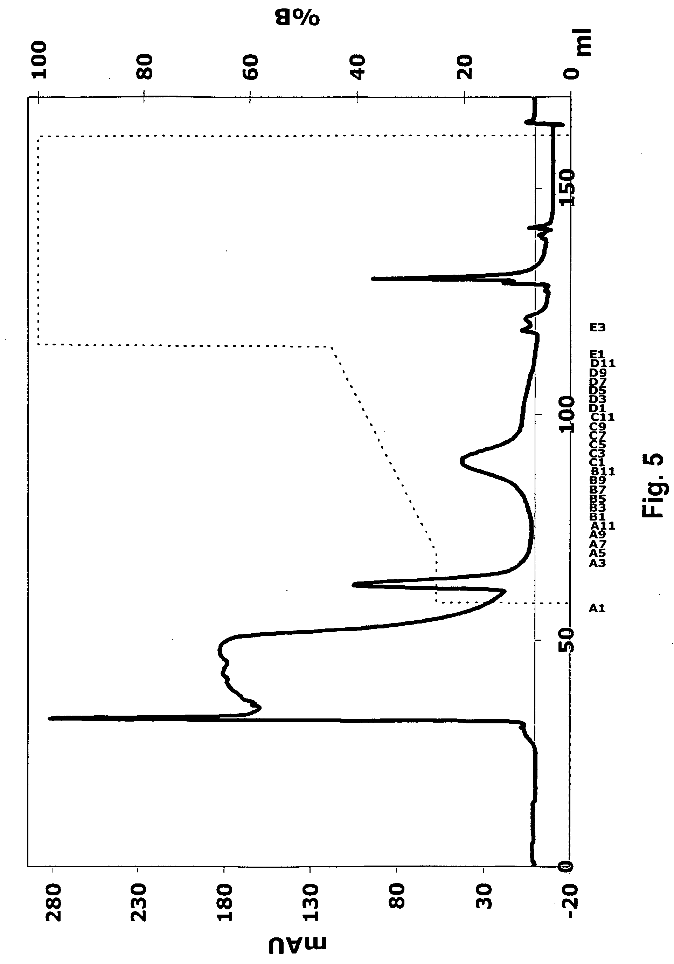

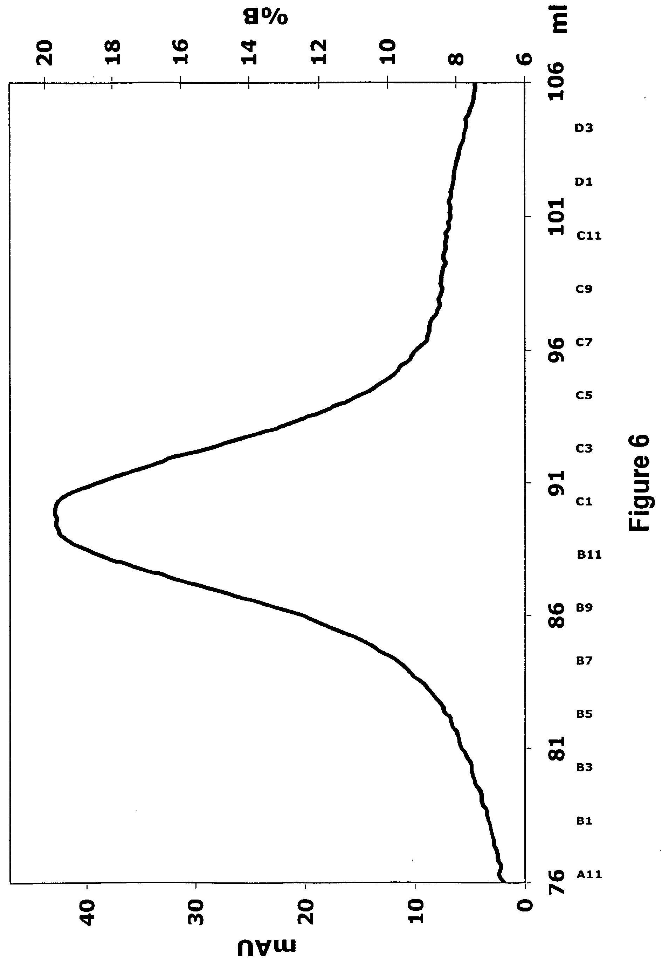

The result of this anion exchange chromatography is shown in Figure 5 and 6.

A pool of fraction C12-D3 was chosen, yielding approx. 1.75 mg/70 %. The Gla content of this pool was compared with that of the original sample used for loading sample above. Gla content analyses of individual fractions were performed using an Agilent HPLC system as described in Example 2.

A comparison of the distribution of Gla before and after separation using anion exchange chromatography as described above is shown in the table below.

| **%-#Gla** | **Before** | **After** | **BeneFIX®, incl. for comparison** |
|---|---|---|---|
| %-#1-8 | 1.0 | 0 | 0 |
| %-#1-9 | 3.3 | 0 | 3 |
| %-#1-10 | 12.7 | 2.3 | 10 |
| %-#1-11 | 32.2 | 25.7 | 27 |
| %-#1-12 | 50.8 | 72.0 | 60 |

Purification using anion exchange chromatography as shown above thus led to the removal of all detectable #1-8- and #1-9-Gla from the original FIX sample. There was also a decrease in the presence of #1-10-Gla and a consequent increase in the proportions of #1-11- and #1-12-Gla.

### EXAMPLE 4: Purification and analysis of different gamma-carboxylated species of FVII

Anion exchange chromatography was used for the separation of different gamma carboxylated forms for recombinant human Factor VIIa (FVII). The method used a SOURCE 15Q column (GE Healthcare) with a bed volume of 1.7 ml, a flow rate of 0.75 ml/min and a temperature of 4°C.

The sample loaded to the column ("loading sample") was of FVII produced in CHO cells. A total of approx. 1.8 mg was loaded. The loading sample was added 50 mM EDTA from a 1 M stock solution and adjusted to pH 8.0 using 0.1 M NaOH.

The buffers used were as follows:
Equilibration buffer: 20 mM Tris/NaOH, pH 8.0
Elution buffer: 20 mM Tris/HCl, 1.5 M Ammonium acetate, pH 8.0
CIP: 1 M NaOH

The anion exchange chromatography procedure was as follows:

| **Step** | **Buffer** | **CV** | | **%-elution buffer** |
|---|---|---|---|---|
| Equilibrate | Equilibration buffer | 5 | | 0% |
| Application | | | | |
| Wash | Equilibration buffer | 5 | | 0% |
| Elution | Elution buffer | 100 | | 0-100 % (gradient) |
| Elution | Elution buffer | 5 | | 100 % (isocratic) |
| CIP1 | NaOH | 5 | | 0% |
| CIP2 | Elution buffer | 10 | | 100% (isocratic) |
| Reequilibrate | Equilibration buffer | 10 | | 0% |

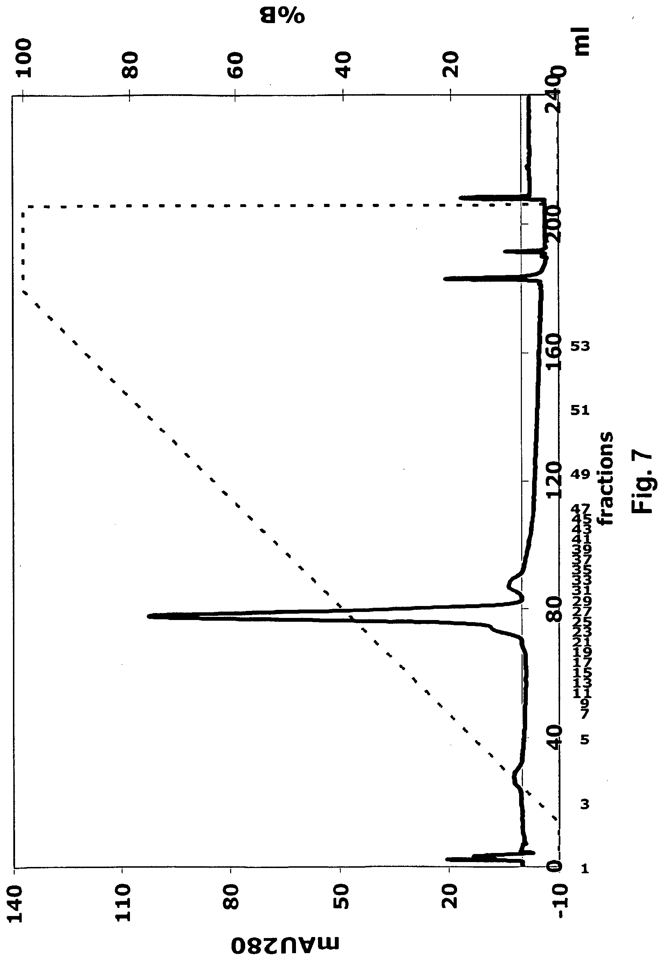

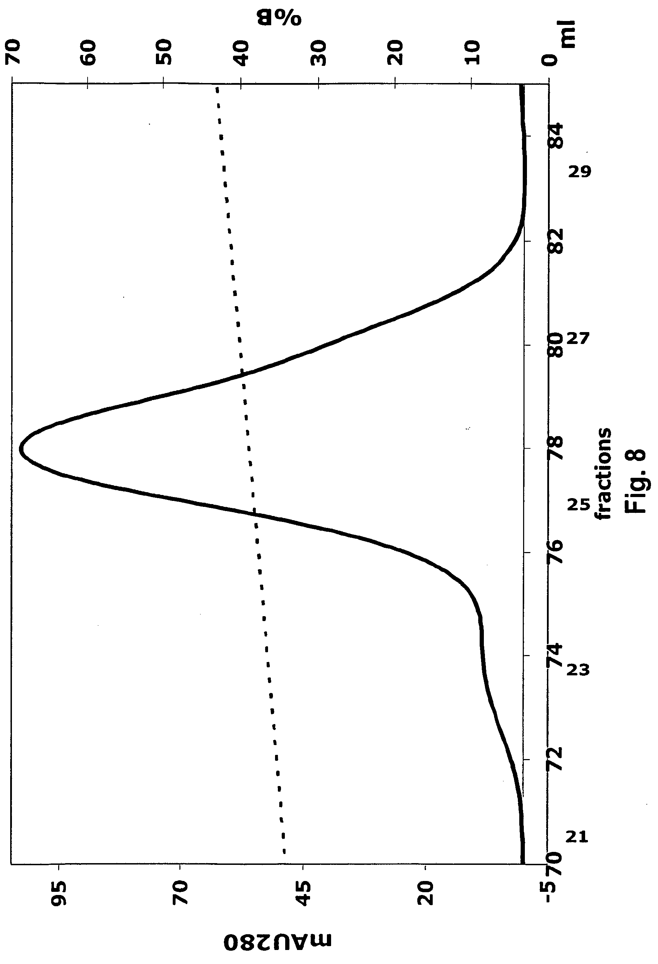

The result of this anion exchange chromatography is shown in Figure 7 and 8.

Gla content analyses of individual fractions (22 to 27) were performed using an Agilent HPLC system as described in Example 2.

A comparison of the distribution of separated FVII Gla species using anion exchange chromatography as described above is shown in the table below.

| **Gla species*** (%) | **Fractions** | | | | | | **Before separation (loading sample)** |
|---|---|---|---|---|---|---|---|
| | **22** | **23** | **24** | **25** | **26** | **27** | |
| **%-#1-8** | 100.0 | 100.0 | 11.0 | - | - | - | < 1 % |
| **%-#1-9** | - | - | 89.0 | 100.0 | 69.2 | 36.7 | 65 |
| **%-#1-10** | - | - | - | - | 30.8 | 63.3 | 34 |

| | | | | | | | |
|---|---|---|---|---|---|---|---|
| * Note that FVII only contains 10 Gla residues in total. | | | | | | | |

Purification using anion exchange chromatography as shown above led to a clear separation of FVIIa Gla species. Thus, a composition of FVII Gla species can be prepared in a fashion preferred by the experimenter.

### EXAMPLE 5: Purification and analysis of different gamma-carboxylated species of FIX

Anion exchange chromatography was used for the separation of different gamma carboxylated forms for recombinant human FIX. The method used a SOURCE 15Q column (GE Healthcare) with a bed volume of 1.7 ml, a flow rate of 0.75 ml/min and a temperature of 4°C.

The sample loaded to the column ("loading sample") was of FIX. A total of approx. 2 mg was loaded. The loading sample was adjusted to pH 8.5 using 0.1 M NaOH.

The buffers used were as follows:
Equilibration buffer: 20 mM Tris/NaOH, pH 8.5
Elution buffer: 20 mM Tris/NaOH, 1.0 M Ammonium chloride, pH 8.5
CIP: 1 M NaOH

The anion exchange chromatography procedure was as follows:

| **Step** | **Buffer** | **CV** | | **%-elution buffer** |
|---|---|---|---|---|
| Equilibrate | Equilibration buffer | 5 | | 0% |
| Application | | | | |
| Wash | Equilibration buffer | 5 | | 0% |
| Elution | Elution buffer | 100 | | 0-100% (gradient) |
| Elution | Elution buffer | 5 | | 100% (isocratic) |
| CIP1 | NaOH | 5 | | 0% |
| CIP2 | Elution buffer | 10 | | 100% (isocratic) |
| Reequilibrate | Equilibration buffer | 10 | | 0% |

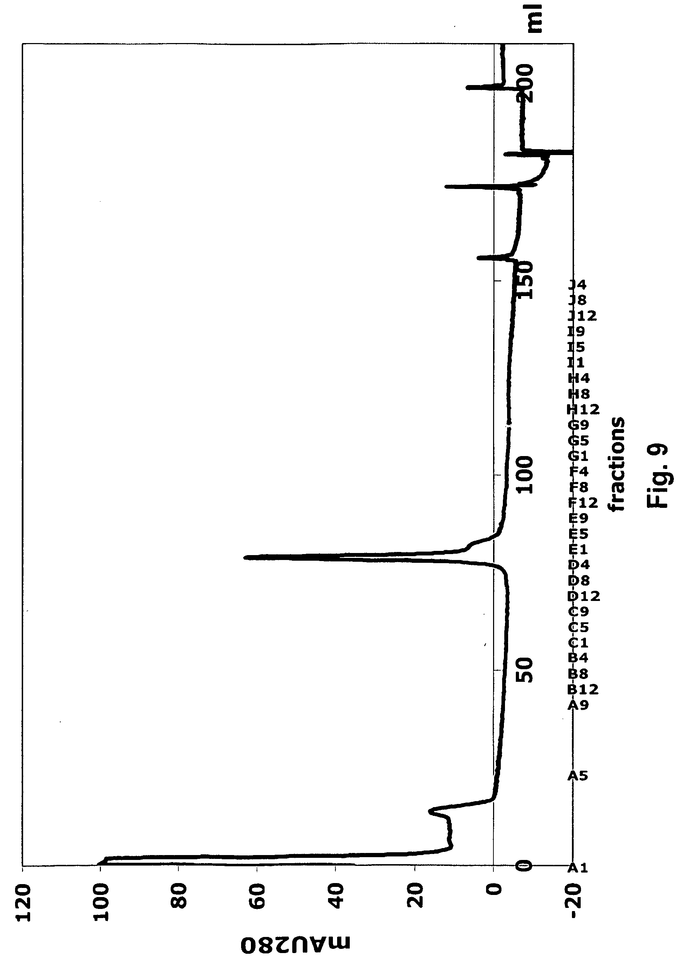

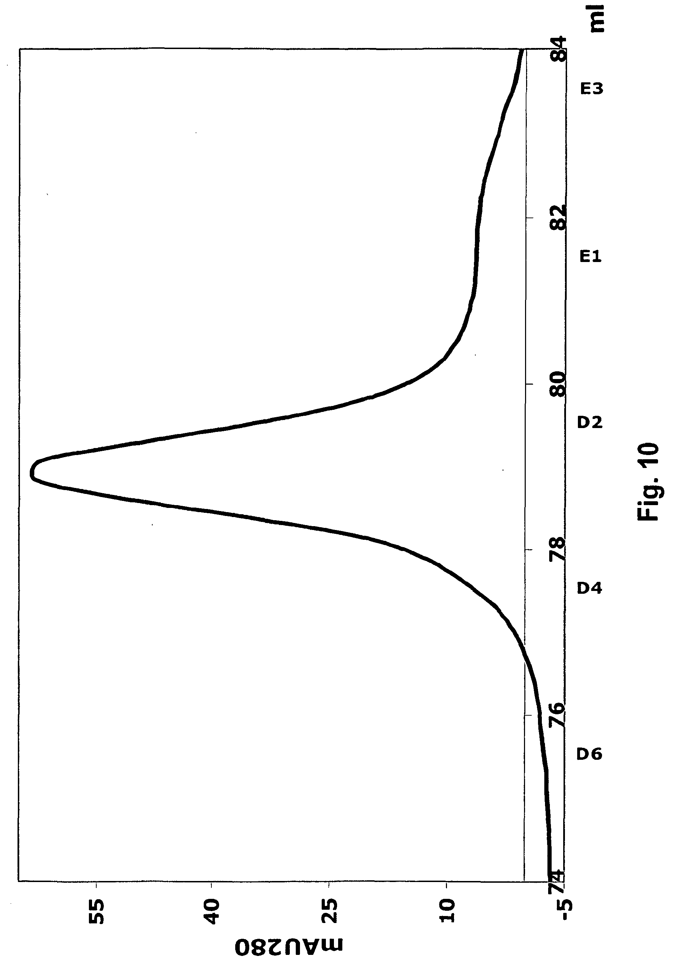

The result of this anion exchange chromatography is shown in Figure 9 and 10.

Gla content analyses of individual fractions (E7 to F12) were performed using an Agilent HPLC system as described in Example 2.

A comparison of the distribution of separated FIX Gla species using anion exchange chromatography as described above is shown in the table below.

| **Gla species (%)** | **Before separation (loading sample)** | **Fractions** | | | | | | | **Benefix®** |
|---|---|---|---|---|---|---|---|---|---|
| | | **E7** | **E8** | **E9** | **E10** | **E11** | **E12** | **F12** | |
| **Gla**#**8** | 1.0 | - | - | - | - | - | - | - | - |
| **Gla#9** | 3.3 | - | - | - | - | - | - | - | 3 |
| **Gla#10** | 12.7 | 18.9 | - | - | - | - | - | - | 10 |
| **Gla#11** | 32.2 | 81.1 | 73.7 | 46.3 | 22.6 | 13.4 | 10.4 | 7.1 | 27 |
| **Gla#12** | 50.8 | - | 26.3 | 53.7 | 77.4 | 86.6 | 89.6 | 92.9 | 60 |

Purification using anion exchange chromatography as shown above led to a clear separation of FIX Gla species. Thus, a composition of FIX Gla species can be prepared in a fashion preferred by the experimenter.

### EXAMPLE 6: Purification and analysis of different gamma-carboxylated species of FIX

Anion exchange chromatography was used for the separation of different gamma carboxylated forms for recombinant human FIX. The method used a SOURCE 15Q column (GE Healthcare) with a bed volume of 1.7 ml, a flow rate of 0.75 ml/min and a temperature of 4°C.

The sample loaded to the column ("loading sample") was of FIX. A total of approx. 2 mg was loaded. The loading sample was adjusted to pH 8.0 using 0.1 M NaOH.

The buffers used were as follows:
Equilibration buffer: 20 mM Tris/NaOH, pH 8.0
Elution buffer: 20 mM Tris/HCl, 1.0 M Sodium acetate, pH 8.0
CIP: 1 M NaOH

The anion exchange chromatography procedure was as follows:

| **Step** | **Buffer** | **CV** | | **%-elution buffer** |
|---|---|---|---|---|
| Equilibrate | Equilibration buffer | 10 | | 0% |
| Application | | | | |
| Wash | Equilibration buffer | 5 | | 0% |
| Elution | Elution buffer | 5 | | 10% (isocratic) |
| Elution | Elution buffer | 40 | | 10-60% (gradient) |
| Elution | Elution buffer | 5 | | 100% (isocratic) |
| CIP1 | NaOH | 5 | | 0% |
| CIP2 | Elution buffer | 10 | | 100% (isocratic) |
| Reequilibrate | Equilibration buffer | 10 | | 0% |

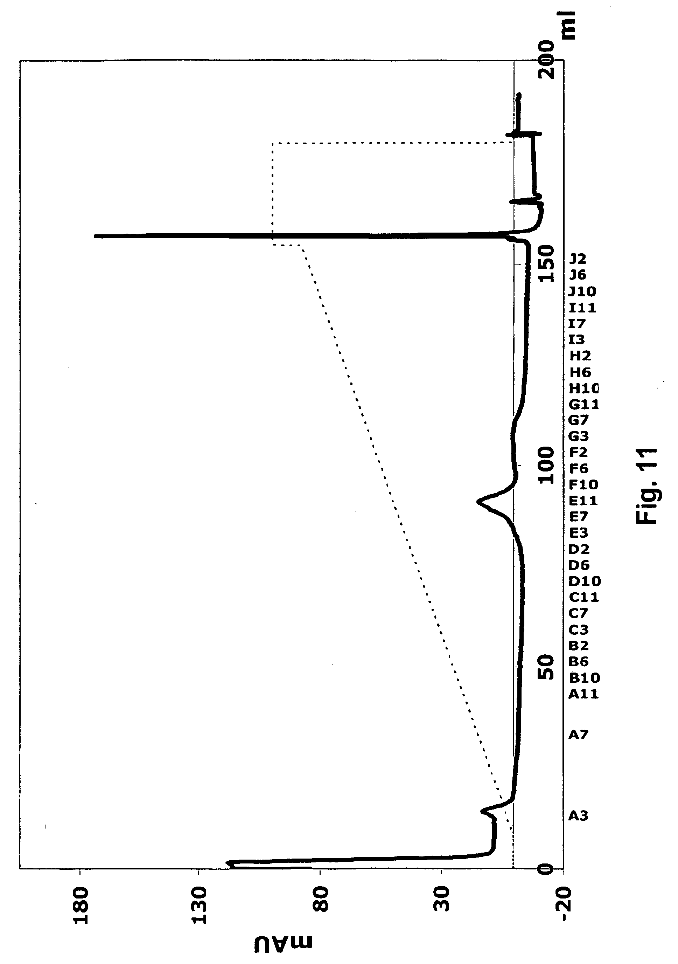

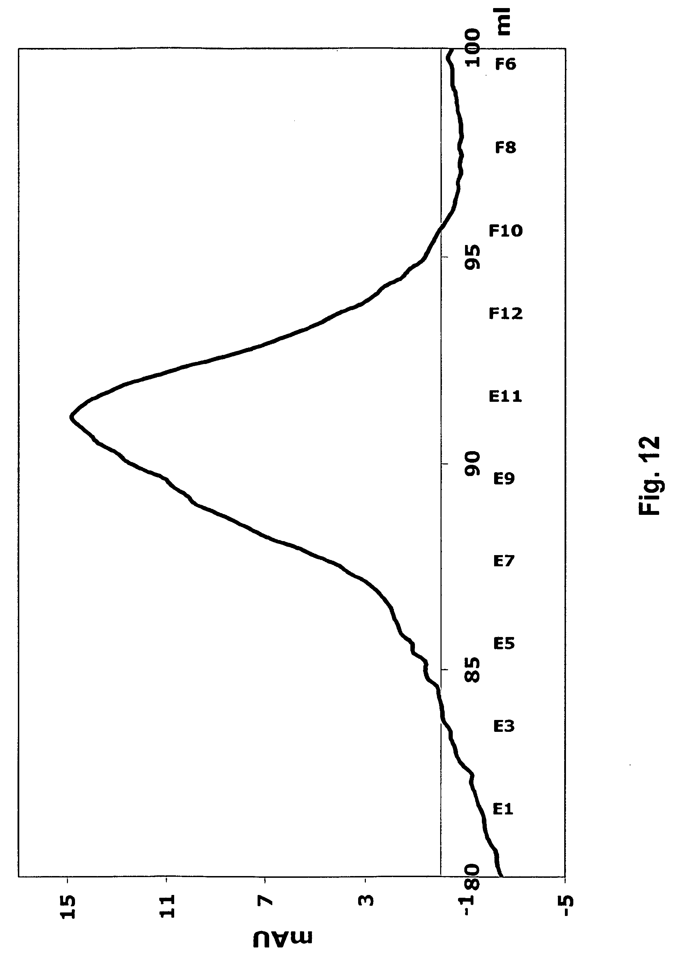

The result of this anion exchange chromatography is shown in Figure 11 and 12.

Gla content analyses of individual fractions (E5 to E1) were performed using an Agilent HPLC system as described in Example 2.

A comparison of the distribution of separated FIX Gla species using anion exchange chromatography as described above is shown in the table below.

| **Gla species (%)** | **Before separation (Application)** | **Fractions** | | | | | | **Benefix®** |
|---|---|---|---|---|---|---|---|---|
| | | **D5** | **D4** | **D3** | **D2** | **D1** | **E1** | |
| **Gla#8** | 1.1 | - | - | - | - | - | - | - |
| **Gla#9** | 2.7 | 16.5 | 3.1 | - | - | - | - | 3 |
| **Gla**#**10** | 12.5 | 49.8 | 27.8 | 6.0 | 5.1 | 4.4 | 5.0 | 10 |
| **Gla#11** | 26.1 | 12.2 | 42.4 | 26.8 | 18.5 | 19.1 | 18.7 | 27 |
| **Gla#12** | 57.5 | 21.5 | 26.8 | 67.2 | 76.4 | 76.5 | 76.4 | 60 |

Purification using anion exchange chromatography as shown above led to some separation of FIX Gla species. Thus, a composition of FIX Gla species can be prepared in a fashion preferred by the experimenter.

### EXAMPLE 7: Purification and analysis of different gamma-carboxylated species of FX

Anion exchange chromatography was used for the separation of different gamma carboxylated forms of a recombinant human FX construct in which the FX activation peptide has been exchanged for a fibrinopeptide A activation peptide (DFLAEGGGVR) and an HPC4 tag added to the C-terminus (DQVDPRLIDGK). The method used a SOURCE 15Q column (GE Healthcare) with a bed volume of 1.7 ml, a flow rate of 0.75 ml/min and a temperature of 4°C.

The sample loaded to the column ("loading sample") was of said FX construct. A total of approx. 2.5 mg was loaded. The loading sample was adjusted to pH 8.0 using 0.1 M NaOH.

The buffers used were as follows:
Equilibration buffer: 20 mM Tris, pH 8.0
Elution buffer: 20 mM Tris/HCl, 1.5 M Ammonium acetate, pH 8.0
CIP: 1 M NaOH

The anion exchange chromatography procedure was as follows:

| **Step** | **Buffer** | **CV** | | **%-elution buffer** |
|---|---|---|---|---|
| Equilibrate | Equilibration buffer | 5 | | 0% |
| Application | | | | |
| Wash | Equilibration buffer | 5 | | 0% |
| Elution | Elution buffer | 100 | | 0-100% (gradient) |
| Elution | Elution buffer | 5 | | 100% (isocratic) |
| CIP1 | NaOH | 5 | | 0% |
| CIP2 | Elution buffer | 10 | | 100% (isocratic) |
| Reequilibrate | Equilibration buffer | 10 | | 0% |

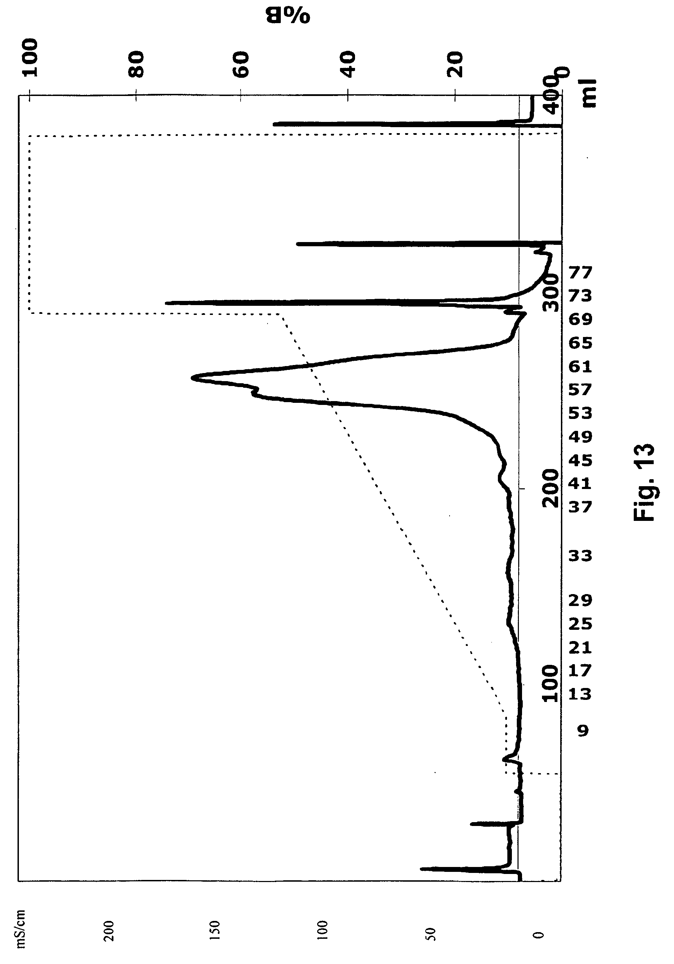

The result of this anion exchange chromatography is shown in Figure 13.

Gla content analyses of individual fractions were performed using an Agilent HPLC system as described in example 2.

Based on the fraction analysis, a comparison of the distribution of separated FX Gla species before and after using the anion exchange chromatography as described above is shown in the table below.

| **Gla species (%)** | **Before separation (loading sample)** | **After separation** | **pdFX** |
|---|---|---|---|
| **Gla#8 and lower** | 3 | - | - |
| **Gla#9** | 30 | - | - |
| **Gla#10** | 42 | 59 | 40 |
| **Gla#11** | 25 | 41 | 43 |

Thus, a composition of the FX construct with an increased amount of Gla#10 and Gla#11 was prepared.

## Claims

1. A method for purifying a polypeptide having a desired content of gamma-carboxyglutamic acid from a sample comprising mixture of species of said polypeptide having different contents of gamma-carboxyglutamic acid, said method comprising the steps of:
(a) loading said sample onto an anion exchange chromatography material;
(b) eluting said polypeptide using a solution at a pH of between 5.0 and 8.5 comprising at least one salt selected from the group consisting of ammonium acetate, ammonium chloride and sodium acetat; and
(c) selecting a fraction obtained from said elution which has an increase in the proportion of #1-10-Gla and/or #1-11-Gla forms of Factor VII or Factor VIIa compared with the proportion of #1-10-Gla and/or #1-11-Gla forms of Factor VII or Factor VIIa in the sample being purified.

2. A method for purifying a polypeptide having a desired content of gamma-carboxyglutamic acid from a sample comprising mixture of species of said polypeptide having different contents of gamma-carboxyglutamic acid, said method comprising the steps of:
(a) loading said sample onto an anion exchange chromatography material;
(b) eluting said polypeptide using a solution at a pH of between 5.0 and 8.5 comprising at least one salt selected from the group consisting of ammonium acetate, ammonium chloride and sodium acetat; and
(c) selecting a fraction obtained from said elution which has an increase in the proportion of #1-11-Gla and/or #1-12-Gla forms of Factor IX compared with the proportion of #1-11-Gla and/or#1-12-Gla forms of Factor IX in the sample being purified.

3. A method according to any one of the preceding claims wherein ammonium acetate, ammonium chloride or sodium acetate is present in the elution buffer at between 0.1 M and 2.0M

4. A method according to claim 3 wherein ammonium acetate, ammonium chloride or sodium acetate is present in the elution buffer at about 0.6 M.

5. A method according to any one of the preceding claims wherein said ion exchange chromatography utilises an equilibration buffer at a pH between 5.0 and 8.5.

## Patentansprüche

1. Verfahren zur Aufreinigung eines Polypeptids mit einem gewünschten Gehalt an gamma-Carboxyglutaminsäure aus einer eine Mischung von Spezies des Polypeptids mit unterschiedlichen Gehalten an gamma-Carboxyglutaminsäure umfassenden Probe, wobei das Verfahren die folgenden Schritte umfasst:
(a) das Aufbringen der Probe auf ein Anionenaustauschchromatographiematerial,
(b) das Eluieren des Polypeptids unter Verwendung einer Lösung mit einem pH-Wert zwischen 5,0 und 8,5, welche mindestens ein aus der aus Ammoniumacetat, Ammoniumchlorid und Natriumacetat bestehenden Gruppe ausgewähltes Salz umfasst, und
(c) die Auswahl einer aus der Elution erhaltenen Fraktion mit einem erhöhten Anteil an #1-10-Gla-und/oder #1-11-Gla-Formen von Faktor VII oder Faktor VIIa im Vergleich zum Anteil an #1-10-Gla- und/oder #1-11-Gla-Formen von Faktor VII bzw. Faktor VIIa in der Probe, die aufgereinigt wird.

2. Verfahren zur Aufreinigung eines Polypeptids mit einem gewünschten Gehalt an gamma-Carboxyglutaminsäure aus einer eine Mischung von Spezies des Polypeptids mit unterschiedlichen Gehalten an gamma-Carboxyglutaminsäure umfassenden Probe, wobei das Verfahren die folgenden Schritte umfasst:
(a) das Aufbringen der Probe auf ein Anionenaustauschchromatographiematerial,
(b) das Eluieren des Polypeptids unter Verwendung einer Lösung mit einem pH-Wert zwischen 5,0 und 8,5, welche mindestens ein aus der aus Ammoniumacetat, Ammoniumchlorid und Natriumacetat bestehenden Gruppe ausgewähltes Salz umfasst, und
(c) die Auswahl einer aus der Elution erhaltenen Fraktion mit einem erhöhten Anteil an #1-11-Gla-und/oder #1-12-Gla-Formen von Faktor IX im Vergleich zum Anteil an #1-11-Gla- und/oder #1-12-Gla-Formen von Faktor IX in der Probe, die aufgereinigt wird.

3. Verfahren nach einem der vorhergehenden Ansprüche, wobei das Ammoniumacetat, Ammoniumchlorid bzw. Natriumacetat im Elutionspuffer in einer Konzentration zwischen 0,1 M und 2,0 M vorliegt.

4. Verfahren nach Anspruch 3, wobei das Ammoniumacetat, Ammoniumchlorid bzw. Natriumacetat im Elutionspuffer in einer Konzentration von etwa 6,0 M vorliegt.

5. Verfahren nach einem der vorhergehenden Ansprüche, wobei bei der Ionenaustauschchromatographie ein Equilibrierungspuffer mit einem pH-Wert zwischen 5,0 und 8,5 verwendet wird.

## Revendications

1. Procédé de purification d'un polypeptide ayant une teneur souhaitée en acide gamma-carboxyglutamique à partir d'un échantillon comprenant un mélange d'espèces dudit polypeptide ayant différentes teneurs en acide gamma-carboxyglutamique, ledit procédé comprenant les étapes de :
(a) chargement dudit échantillon sur un matériau de chromatographie d'échange d'anions ;
(b) élution dudit polypeptide au moyen d'une solution à un pH compris entre 5,0 et 8,5 comprenant au moins un sel choisi dans le groupe constitué de l'acétate d'ammonium, le chlorure d'ammonium et l'acétate de sodium ; et
(c) sélection d'une fraction obtenue à partir de ladite élution qui a une augmentation de la proportion de formes #1-10-Gla et/ou #1-11-Gla de facteur VII ou facteur VIIa par rapport à la proportion de formes #1-10-Gla et/ou #1-11-Gla de facteur VII ou facteur VIIa dans l'échantillon étant purifié.

2. Procédé de purification d'un polypeptide ayant une teneur souhaitée en acide gamma-carboxyglutamique à partir d'un échantillon comprenant un mélange d'espèces dudit polypeptide ayant différentes teneurs en acide gamma-carboxyglutamique, ledit procédé comprenant les étapes de :
(a) chargement dudit échantillon sur un matériau de chromatographie d'échange d'anions ;
(b) élution dudit polypeptide au moyen d'une solution à un pH compris entre 5,0 et 8,5 comprenant au moins un sel choisi dans le groupe constitué de l'acétate d'ammonium, le chlorure d'ammonium et l'acétate de sodium ; et
(c) sélection d'une fraction obtenue à partir de ladite élution qui a une augmentation de la proportion de formes #1-11-Gla et/ou #1-12-Gla de facteur IX par rapport à la proportion de formes #1-11-Gla et/ou #1-12-Gla de facteur IX dans l'échantillon étant purifié.

3. Procédé selon l'une quelconque des revendications précédentes dans lequel l'acétate d'ammonium, le chlorure d'ammonium ou l'acétate de sodium est présent dans le tampon d'élution entre 0,1 M et 2,0 M.

4. Procédé selon la revendication 3 dans lequel le chlorure d'ammonium ou l'acétate de sodium est présent dans le tampon d'élution à environ 0,6 M.

5. Procédé selon l'une quelconque des revendications précédentes dans lequel ladite chromatographie d'échange d'ions utilise un tampon d'équilibrage à un pH compris entre 5,0 et 8,5.
